# EUROPEAN PATENT SPECIFICATION

(11) **EP 2 361 564 B1**
(45) Date of publication and mention of the grant of the patent: **19.12.2012**
(21) Application number: 10187212.5
(22) Date of filing: 12.10.2010
(51) Int. Cl.: A61B 17/28, A61B 17/29, A61B 19/00, A61B 8/12

(54) **Forceps for pathological diagnosis**
Zange zur pathologischen Diagnose
Pinces pour le diagnostic de pathologies

(30) Priority: 23.02.2010 TW 099105152
(43) Date of publication of application: 31.08.2011
(73) Proprietor: Chi Mei Medical Center, Tainan (TW)
(72) Inventor: Su, Ying-Chieh, Beimen Rd., Tainan City (TW)
(74) Representative: Cohausz & Florack

(56) References cited:
- WO-A2-02/096501
- WO-A2-2004/069025
- US-A1- 2003 120 306
- US-A1- 2007 213 766
- US-B1- 6 183 484
- US-B1- 6 371 973

## Description

The invention relates to a forceps, more particularly to a forceps for pathological diagnosis

Cancers, particularly lung cancer, are fatal diseases. Early diagnosis is therefore of extreme importance. Governments, medical centers and medical equipment manufacturers are always looking for new and effective methods and apparatuses for diagnosing and treating cancers.

During diagnosis of pathological changes in the lung, a common procedure is to first locate the pathological change through radiographs or via computerized tomography (CT) scan images, and then perform excision surgery. However, when the area of pathological change is small, it is hard to identify the precise location thereof with the naked eye under videothoracoscopic guide during surgery. In this kind of situation, a surgeon needs to refer to the radiographs or CAT scan images and utilize his/her sense of touch to grope for and feel the area of pathological change. Biopsy has to be performed to evaluate whether the surgeon has correctly located the area of pathological change before the actual excision can be performed. A surgeon's clinical experience is heavily relied upon under such circumstance, and it might be time-consuming even for an experienced surgeon. Moreover, there is still the possibility of making a wrong judgment when no other technological assistance can be provided to the surgeon. This greatly increases a patient's risks during surgery, and lengthens operation time.

Therefore, the object of the present invention is to provide a forceps that is convenient for pathological diagnosis of organs.

WO 02/096501 A2 on which the preamble of claim 1 is based discloses a forceps for pathological diagnosis adapted to clamp a target tissue for diagnosis of pathological change in the target tissue.

According to the present invention, there is provided a forceps for pathological diagnosis. The forceps is adapted to clamp a target tissue for diagnosis of pathological change in the target tissue, and includes first and second proximate arm segments, first and second distal arm segments, a coupling member, an ultrasonic probe and a shielding member.

Each of the first and second proximate arm segments has a finger-operable grip end and a hinging-side end opposite to each other. The hinging-side ends of the proximate arm segments are hinged to each other such that the finger-operable grip ends of the first and second proximate arm segments are movable towards or away from each other about a first hinge axis.

Each of the first and second distal arm segments has a grasping-jaw end and a hinged-side end opposite to each other. The hinged-side ends of the first and second distal arm segments are hinged to each other such that the grasping-jaw ends of the first and second distal arm segments are movable towards or away from each other about a second hinge axis parallel to the first hinge axis. The grasping-jaw ends are adapted for clamping the target tissue therebetween when moved towards each other about the second hinge axis.

The coupling member is disposed to couple the hinged-side ends with the hinging-side ends so as to bring the relative movement between the first and second proximate arm segments into synchronization with that between the first and second distal arm segments.

The ultrasonic probe is mounted to the grasping-jaw end of the first distal arm segment, and is adapted to transmit ultrasonic waves towards the target tissue and to receive reflected ultrasonic waves traveling from the target tissue.

The shielding member is disposed on the grasping-jaw end of the second distal arm segment, and is capable of partially reflecting ultrasonic waves transmitting through the target tissue.

Other features and advantages of the present invention will become apparent in the following detailed description of the preferred embodiments with reference to the accompanying drawings, of which:
Figure 1 is an exploded schematic view of the first preferred embodiment of a forceps according to the present invention;
Figure 2 is an assembled schematic view of the first preferred embodiment, illustrating grasping-jaw ends of first and second distal segments being moved away from each other;
Figure 3 is an assembled schematic view of the first preferred embodiment, illustrating the grasping-jaw ends of the first and second distal segments being moved toward each other so as to clamp a target tissue therebetween;
Figure 4 is a partially exploded schematic view of the second preferred embodiment of a forceps according to the present invention;
Figure 5 is an assembled schematic view of the second preferred embodiment; and
Figure 6 is an assembled schematic view of the third preferred embodiment of a forceps according to the present invention.

Before the present invention is described in greater detail, it should be noted that like elements are denoted by the same reference numerals throughout the disclosure.

It should be noted herein that the relative proportions and dimensions of the elements shown in the drawings are for illustrative purposes only, and should not be conceived to limit the scope of the present invention.

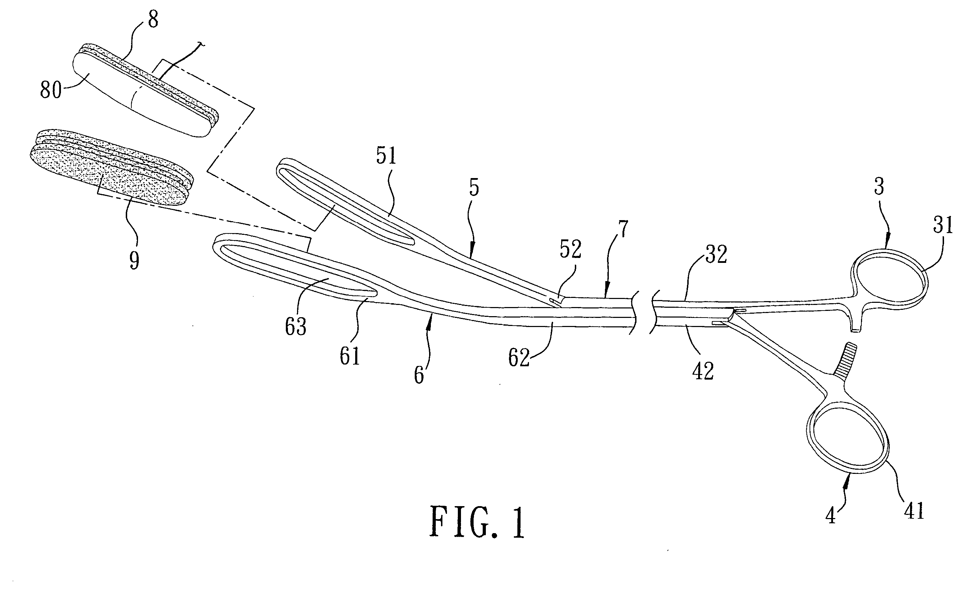

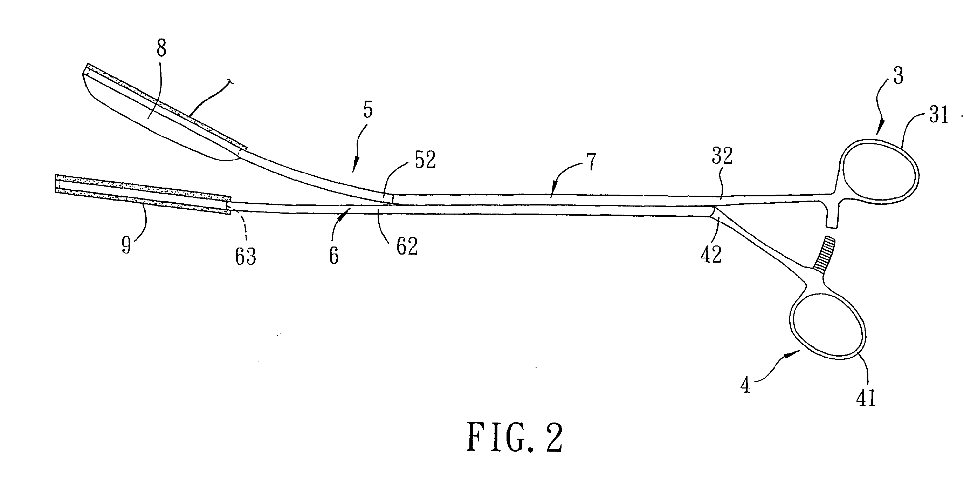

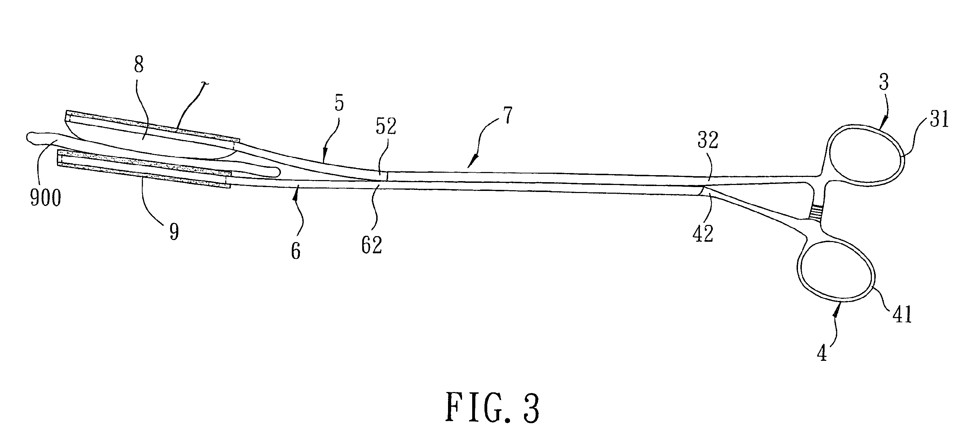

With reference to Figures 1, 2 and 3, the first preferred embodiment of a forceps for pathological diagnosis according to the present invention is adapted to clamp a target tissue 900 (only shown in Figure 3) for diagnosis of pathological change in the target tissue 900, especially during surgical operations where it is necessary to locate the pathological change.

The forceps includes a first proximate arm segment 3, a second proximate arm segment 4, a first distal arm segment 5, a second distal arm segment 6, a coupling member 7, an ultrasonic probe 8, and a shielding member 9. In this embodiment, the forceps is shown to be an elongated forceps particularly used for microsurgery or endoscopic surgery.

Each of the first and second proximate arm segments 3, 4 has a finger-operable grip end 31, 41 and a hinging-side end 32, 42 opposite to each other. The hinging-side ends 32, 42 of the first and second proximate arm segments 3, 4 are hinged to each other such that the finger-operable grip ends 31, 41 of the first and second proximate arm segments 3, 4 are movable towards or away from each other about a first hinge axis.

Each of the first and second distal arm segments 5, 6 has a grasping-jaw end 51, 61 and a hinged-side end 52, 62 opposite to each other. The hinged-side ends 52, 62 of the first and second distal arm segments 5, 6 are hinged to each other such that the grasping-jaw ends 51, 61 of the first and second distal arm segments 5, 6 are movable towards or away from each other about a second hinge axis parallel to the first hinge axis. The grasping-jaw ends 51, 61 are adapted for clamping the target tissue 900 therebetween when moved towards each other about the second hinge axis.

The coupling member 7 is disposed to couple the hinged-side ends 32, 42 of the first and second proximate arm segments 3, 4 with the hinging-side ends 52, 62 of the first and second distal arm segments 5, 6 so as to bring the relative movement between the first and second proximate arm segments 3, 4 into synchronization with that between the first and second distal arm segments 5, 6. In this embodiment, the coupling member 7 includes a couplingmechanism (not shown) that brings the relative movement between the first and second proximate arm segments 3, 4 into synchronization with that between the first and second distal arm segments 5, 6. It should be noted herein that since the feature of this invention does not reside in the translation of movement described above, further details are omitted herein for the sake of brevity.

The ultrasonic probe 8 is mounted to the grasping-jaw end 51 of the first distal arm segment 5, and is adapted to transmit ultrasonic waves towards the target tissue 900 and to receive reflected ultrasonic waves traveling from the target tissue 900. The ultrasonic probe 8 is adapted to be connected to an ultrasonic imaging device (not shown) so as to generate an ultrasonic image with reference to the ultrasonic waves received as a result of the traveling of the reflected ultrasonic waves from the target tissue 900.

The shielding member 9 is disposed on the grasping-jaw end 61 of the second distal arm segment 6, and is capable of partially reflecting ultrasonic waves transmitting through the target tissue 900. The shielding member 9 is made from a biocompatible material, such as silicone gel, whose density permits partial reflection of the ultrasonic waves transmitting through the target tissue 900.

During use, a surgeon may look at a radiograph or a computerized axial tomography (CAT) scan image to get a general picture of where a pathological change might be located, and then use the grasping-jaw ends 51, 61 of the first and second distal arm segments 5, 6 to clamp the target tissue 900 therebetween, such that when the ultrasonic probe 8 is activated (by an ultrasonic equipment (not shown)) (i.e., ultrasonic waves are transmitted toward the target tissue 900), ultrasonic waves received by the ultrasonic probe 8 as a result of the traveling of the reflected ultrasonic waves from the target tissue 900 can be used to form an ultrasonic image by the ultrasonic imaging device (not shown) for reference by the surgeon to diagnose where the pathological change is located. Since the amount of ultrasonic waves reflected from the target tissue 900 differs as the density of the target tissue 900 varies, and since the density of a pathologically-changed portion of an organ is significantly different from that of a normal portion of the same organ, the ultrasonic image of the target tissue 900 clamped by the grasping-jaw ends 51, 61 of the first and second distal arm segments 5, 6 can be utilized for diagnosis. The surgeon can then perform excision surgery on the correct portion of the organ.

The ultrasonic probe 8 and the shielding member 9 are to be cleansed and sterilized for future use.

In this embodiment, the grasping-jaw end 61 of the second distal arm segment 6 is formed with a mounting hole 63, and the shielding member 9 is configured to be fitted in the mounting hole 63. The mounting hole 63 is formed because without it, an enormous amount of ultrasonic signals will reflect off the face of the grasping-jaw end 61 (which is made of metal), causing a bright spot in the resulting ultrasonic image that obstruct diagnosis. The shielding member 9 is provided to permit thereat partial reflection of the ultrasonic waves transmitting through the target tissue 900 so as to ensure that the resulting ultrasonic image will not be insufficiently clear for proper diagnosis.

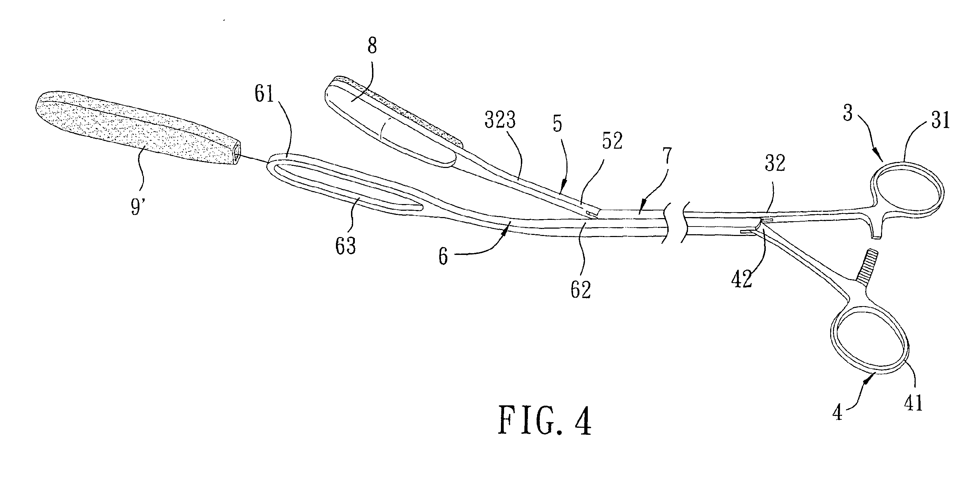

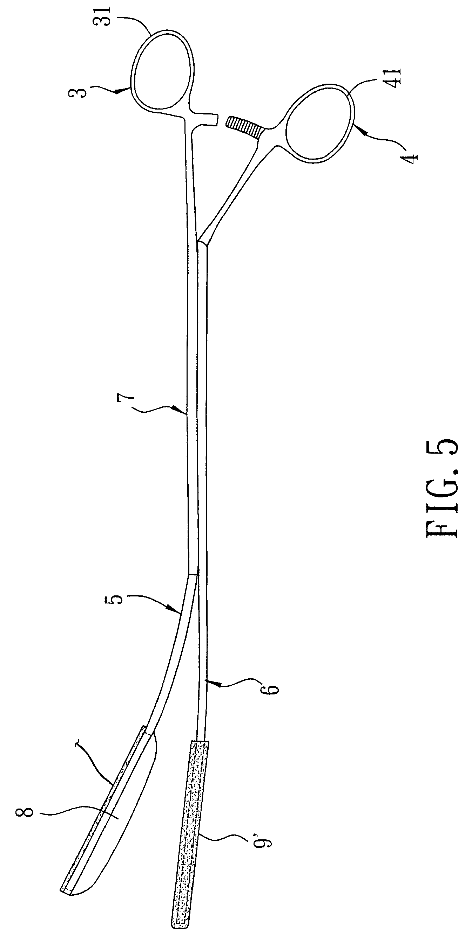

With reference to Figure 4 and Figure 5, the second preferred embodiment of a forceps according to the present invention differs from the first preferred embodiment in that the shielding member 9' of the second preferred embodiment is in the form of a covering that covers the grasping-jaw end 61 of the second distal arm segment 6.

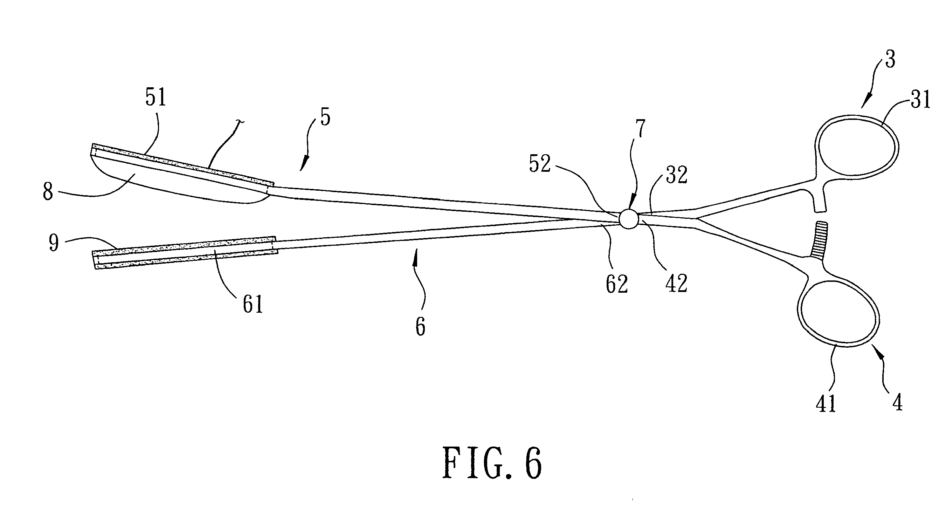

With reference to Figure 6, the third preferred embodiment of a forceps according to the present invention differs from the previous embodiments in that the coupling member 7 is configured so as to bring the first axis in alignment with the second hinge axis. In addition, the hinging-side end 32, 42 of each of the first and second proximate arm segments 3, 4 is integrally formed with the hinged-side end 62, 52 of a corresponding one of the first and second distal arm segments 6, 5. In other words, the third embodiment operates in a way similar to a pair of scissors.

In summary, through the structural configuration of the grasping-jaw ends 51, 61 of the first and second distal arm segments 5, 6, and the provision of the ultrasonic probe 8 and the shielding member 9, the forceps of the present invention is adapted to clamp a target tissue 900, and to facilitate diagnosis of pathological change in the target tissue 900 by a surgeon so as to assist the surgeon to quickly locate the areas of the pathological change, and to reduce the time of surgery to thereby reduce a patient's risks during the surgery.

## Claims

1. A forceps for pathological diagnosis adapted to clamp a target tissue (900) for diagnosis of pathological change in the target tissue (900), said forceps comprising:
first and second proximate arm segments (3, 4) each having a finger-operable grip end (31, 41) and a hinging-side end (32, 42) opposite to each other, said hinging-side ends (32, 42) of said first and second proximate arm segments (3, 4) being hinged to each other such that said finger-operable grip ends (31, 41) of said first and second proximate arm segments (3, 4) are movable towards or away from each other about a first hinge axis;
first and second distal arm segments (5, 6) each having a grasping-jaw end (51, 61) and a hinged-side end (52, 62) opposite to each other;
an ultrasonic probe (8) mounted to said grasping-jaw end (51) of said first distal arm segment (5), and adapted to transmit ultrasonic waves towards the target tissue (900) and to receive reflected ultrasonic waves traveling from the target tissue (900); and
a shielding member (9) disposed on said grasping-jaw end (61) of said second distal arm segment (6), and capable of partially reflecting ultrasonic waves transmitting through the target tissue (900),
**characterized in that** said grasping-jaw end (61) of said second distal arm segment (6) is formed with a mounting hole (63) therein, and **in that** said hinged-side ends (52, 62) of said first and second distal arm segments (5, 6) are hinged to each other such that said grasping-jaw ends (51, 61) of said first and second distal arm segments (5, 6) are movable towards or away from each other about a second hinge axis parallel to the first hinge axis, said grasping-jaw ends (51, 61) being adapted for clamping the target tissue (900) therebetween when moved towards each other about the second hinge axis, wherein the forceps comprises a coupling member (7) disposed to couple said hinged-side ends (32, 42) with said hinging-side ends (52, 62) so as to bring the relative movement between said first and second proximate arm segments (3, 4) into synchronization with that between said first and second distal arm segments (5, 6).

2. The forceps as claimed in Claim 1, **characterized in that** said shielding member (9) is configured to be fitted in said mounting hole (63).

3. The forceps as claimed in Claim 1, **characterized in that** said shielding member (9') is in the form of a covering that covers said grasping-jaw end (61) of said second distal arm segment (6).

4. The forceps as claimed in Claim 1, **characterized in that** said ultrasonic probe (8) is adapted to be connected to an ultrasonic imaging device so as to generate an ultrasonic image with reference to the ultrasonic waves received as a result of the traveling of the reflected ultrasonic waves from the target tissue (900).

5. The forceps as claimed in Claim 1, **characterized in that** said shielding member (9) is made from a biocompatible material.

6. The forceps as claimed in Claim 1, wherein said coupling member (7) is configured so as to bring said first axis in alignment with said second hinge axis.

7. The forceps as claimed in Claim 1, **characterized in that** said hinging-side end (32, 42) of each of said first and second proximate arm segments (3, 4) is integrally formed with said hinged-side end (62, 52) of a corresponding one of said first and second distal arm segments (6, 5).

## Patentansprüche

1. Pathologische Diagnosezange, die dazu geeignet ist, um ein Zielgewebe (900) zur Diagnose einer pathologischen Änderung in dem Zielgewebe (900) einzuklemmen, wobei die Zange aufweist:
erste und zweite proximale Armsegmente (3, 4), die jeweils ein mit den Fingern zu betätigendes Griffende (31, 41) und ein gelenkseitiges Ende (32, 42), die einander gegenüberliegen, aufweisen, wobei die gelenkseitigen Enden (32, 42) der ersten und zweiten proximalen Armsegmente (3, 4) zueinander derart angelenkt sind, dass die mit den Fingern zu betätigenden Griffenden (31, 41) der ersten und zweiten proximalen Armsegmente (3, 4) um eine erste Gelenkachse aufeinander zu oder voneinander weg bewegbar sind;
erste und zweite distale Armsegmente (5, 6), die jeweils ein Greifbackenende (51, 61) und ein gelenkseitiges Ende (52, 62), die einander gegenüberliegen, aufweisen;
eine Ultraschallsonde (8), die an dem Greifbackenende (51) des ersten distalen Armsegments (5) angebracht ist und dazu geeignet ist, um Ultraschallwellen in Richtung auf das Zielgewebe (900) zu übertragen und um reflektierte Ultraschallwellen, die von dem Zielgewebe (900) ausgehen, zu empfangen; und
ein Abschirmelement (9), das an dem Greifbackenende (61) des zweiten distalen Armsegments (6) angeordnet ist und in der Lage ist, Ultraschallwellen, die durch das Zielgewebe (900) übertragen werden, teilweise zu reflektieren,
**dadurch gekennzeichnet, dass** das Greifbackenende (61) des zweiten distalen Armsegments (6) mit einem Montageloch (63) darin gebildet ist, und dass die gelenkseitigen Enden (52, 62) der ersten und zweiten distalen Armsegmente (5, 6) aneinander derart angelenkt sind, dass die Greifbackenenden (51, 61) der ersten und zweiten distalen Armsegmente (5, 6) um eine zweite Gelenkachse herum, die zu der ersten Gelenkachse parallel ist, aufeinander zu und voneinander weg bewegbar sind, wobei die Greifbackenenden (51, 61) dazu geeignet sind, das Zielgewebe (900) dazwischen einzuklemmen, wenn sie um die zweite Gelenkachse herum aufeinander zu bewegt werden, wobei die Zange ein Koppelelement (7) aufweist, das angeordnet ist, um die gelenkseitigen Enden (32, 42) mit den gelenkseitigen Enden (52, 62) zu koppeln, um die relative Bewegung zwischen den ersten und zweiten proximalen Armsegmenten (3, 4) mit derjenigen zwischen den ersten und zweiten distalen Armsegmenten (5, 6) zu synchronisieren.

2. Zange nach Anspruch 1, **dadurch gekennzeichnet, dass** das Abschirmelement (9) konfiguriert ist, um in das Montageloch (63) zu passen.

3. Zange nach Anspruch 1, **dadurch gekennzeichnet, dass** das Abschirmelement (9') in Form einer Abdeckung vorliegt, die das Greifbackenende (61) des zweiten distalen Armsegments (6) abdeckt.

4. Zange nach Anspruch 1, **dadurch gekennzeichnet, dass** die Ultraschallsonde (8) dazu geeignet ist, um an eine Ultraschall-Bildgebungsvorrichtung angeschlossen zu werden, um ein Ultraschallbild mit Bezug auf die Ultraschallwellen zu generieren, die als Ergebnis der Ausbreitung der reflektierten Ultraschallwellen aus dem Zielgewebe (900) empfangen werden.

5. Zange nach Anspruch 1, **dadurch gekennzeichnet, dass** das Abschirmelement (9) aus einem biokompatiblen Material hergestellt ist.

6. Zange nach Anspruch 1, wobei das Koppelelement (7) konfiguriert ist, um die erste Achse auf die zweite Gelenkachse auszurichten.

7. Zange nach Anspruch 1, **dadurch gekennzeichnet, dass** das gelenkseitige Ende (32, 42) jedes der ersten und zweiten proximalen Armsegmente (3, 4) mit dem gelenkseitigen Ende (62, 52) eines entsprechenden der ersten und zweiten distalen Armsegmente (6, 5) einstückig gebildet ist.

## Revendications

1. Forceps pour diagnostic pathologique adapté pour serrer un tissu cible (900) pour le diagnostic de modification pathologique dans le tissu cible (900), ledit forceps comprenant:
un premier et un deuxième segment de bras proches (3,4) ayant chacun une extrémité de saisie pouvant être utilisé avec les doigts (31,41) et une extrémité de charnière (32,42) opposées l'une à l'autre, lesdites extrémités de charnière (32,42) desdits premier et deuxième segments de bras proches (3,4) étant dépendantes l'une de l'autre de sorte que lesdites extrémités de saisie pouvant être utilisées avec les doigts (31,41) desdits premier et deuxième segments de bras proches (3,4) soient déplaçables l'une vers l'autre ou loin l'une de l'autre autour d'un premier axe de charnière ;
un premier et un deuxième segment de bras distaux (5,6) ayant chacun une extrémité de pince de saisie (51,61) et une extrémité de charnière (52,62) opposées l'une à l'autre ;
une sonde à ultrasons (8) montée sur ladite extrémité de pince de saisie (51) dudit premier segment de bras distal (5), et adapté pour transmettre des ondes ultrasonores vers le tissu cible (900) et pour recevoir des ondes ultrasonores réfléchies provenant du tissu cible (900); et un élément de blindage (9) disposé sur ladite extrémité de pince de saisie (61) dudit deuxième segment de bras distal (6), et capable de refléter partiellement des ondes ultrasonores en transmettant par le tissu cible (900),
**caractérisé en ce que** ladite extrémité de pince de saisie (61) dudit deuxième segment de bras distal (6) est formée d'un trou de montage (63) à l'intérieur, et **en ce que** lesdites extrémités de charnière (52,62) desdits premier et deuxième segments de bras distaux (5,6) sont dépendantes l'une de l'autre de sorte que lesdites extrémités de saisie (51,61) desdits premier et deuxième segments de bras distaux (5,6) soient déplaçables l'une vers l'autre ou loin l'une de l'autre autour d'un deuxième axe de charnière parallèle au premier axe de charnière, lesdites extrémités de saisie (51,61) étant adaptées pour serrer le tissu cible (900) entre elles quand elles sont déplacées l'une vers l'autre autour du deuxième axe de charnière, où le forceps comprend un élément de couplage (7) disposé pour accoupler lesdites extrémités de charnière (32,42) avec lesdites extrémités de charnière (52,62) de manière à amener le mouvement relatif entre lesdits premier et deuxième segments de bras proches (3,4) en synchronisation entre ces dits premier et deuxième segments de bras distaux (5,6).

2. Forceps tel que revendiqué à la Revendication 1, **caractérisé en ce que** ledit élément de blindage (9) est configuré pour être placé sur ledit trou de montage (63).

3. Forceps tel que revendiqué à la Revendication 1, **caractérisé en ce que** ledit élément de blindage (9') est de la forme d'un couvercle qui couvre ladite extrémité de pince de saisie (61) dudit deuxième segment de bras distal (6).

4. Forceps tel que revendiqué à la Revendication 1, **caractérisé en ce que** ladite sonde ultrasonore (8) est adaptée pour être raccordée à un dispositif d'imagerie acoustique pour générer une image acoustique avec référence aux ondes ultrasonores reçues en résultat du déplacement des ondes ultrasonores réfléchies provenant du tissu cible (900).

5. Forceps tel que revendiqué à la Revendication 1, **caractérisé en ce que** ledit élément de blindage (9) est fait d'un matériau biocompatible.

6. Forceps tel que revendiqué à la Revendication 1, où ledit élément de couplage (7) est configuré de manière à amener le premier axe dans l'alignement dudit deuxième axe de charnière.

7. Forceps tel que revendiqué à la Revendication 1, **caractérisé en ce que** ladite extrémité de charnière (32,42) de chacun desdits premier et deuxième segments de bras proches (3, 4) est intégralement formée de ladite extrémité de charnière (62,52) d'un segment correspondant des dits premier et deuxième segments de bras distaux (6,5).
